# EUROPEAN PATENT APPLICATION

(11) **EP 2 387 992 A1**
(43) Date of publication of application: **23.11.2011**
(21) Application number: 11005053.1
(22) Date of filing: 29.06.2007
(51) Int. Cl.: A61K 9/16, A61K 31/366

(54) **Improvements relating to nanodispersions**

(30) Priority: 13.07.2006 GB 0613925
(62) Divisional of application: 07786931.1
(71) Applicant: Iota NanoSolutions Limited, London, EC4Y 0DY (GB)
(72) Inventor: Angus, Doris, Liverpool, L69 7ZB (GB); Duncalf, David John, Liverpool, L69 7ZB (GB); Foster, Alison Jayne, Liverpool, L69 7ZB (GB); Long, James, Liverpool, L69 7ZB (GB); Rannard, Steven Paul, Liverpool, L69 7ZB (GB); Wang, Dong, Liverpool, L69 7ZB (GB); Elphick, James Andrew, Oxford, OX29 8RA (GB)
(74) Representative: Cawley, Aimee Elizabeth

(57) **Abstract**

The invention provides process for making water-soluble nano-dispersions of water-insoluble materials in a water-soluble carrier material comprising the steps of:
(i) providing a single phase mixture of:
(a) a non-aqueous solvent or a mixture of miscible non-aqueous solvents,
(b) at least one carrier material soluble in non-aqueous solvent (a), said carrier material being also soluble in water and solid at ambient temperature,
(c) at least one water-insoluble payload material which is soluble in non-solvent (a), and,

(ii) drying the mixture to remove solvent (a) and thereby obtain the carrier material (b) in solid form with payload (c) dispersed therein as nanoparticles,

wherein the product of the drying step is water dispersible to give an aqueous solution of (b) and an aqueous nano-dispersion of (c).

## Description

### Technical Field

The present invention relates to dried compositions which form so-called 'nano dispersions' when placed in water or other solvents and deliver a material which is not normally soluble in water or said other solvent.

### Background to the Invention

Many potentially useful materials are insoluble or at best only sparingly soluble in water. This places significant limits on their usefulness or requires that they are used in combination with solvents which almost always more expensive than water and which may have physiological incompatibilities or environmental negatives.

Our co-pending applications including GB 0501835.3 relate to the use of emulsions of immiscible solvents to prepare a poorly soluble material in a form that is rapidly dispersible. In the above-mentioned applications a `carrier' material (such as a surfactant or a polymer) is dissolved in an aqueous solvent (typically water) and a water-insoluble "payload' material is dissolved in a non-aqueous solvent. An emulsion of the aqueous and non-aqueous solutions is prepared and then dried, such as by freeze or spray drying, to form a material which is believed to comprise a nano-scale dispersion of an insoluble 'payload' material in a soluble matrix formed from the 'carrier' material.

A similar approach can be used to provide oil-insoluble payload materials which are rapidly dispersible in oils and other non-aqueous solvents by forming a nano-dispersion of these payload materials in an oil-soluble carrier.

When the dried powder material obtained from the emulsion is placed in water, the matrix of carrier material dissolves releasing the nano-disperse payload material. This forms a solution-like 'nano-dispersion' of the supposedly water-insoluble payload material.

For ease of reference the payload and the carrier are referred to in the present specification as 'contra-soluble' where one is soluble in water (or aqueous solution) and the other is effectively insoluble in water (or aqueous solution) but is soluble in some at least partially non-aqueous solvent.

There is a need to improve upon this process by simplifying the formation of the emulsion and the drying step.

WO 2005/011636 discloses a non-emulsion based spray drying process for forming 'solid amorphous dispersions' of drugs in polymers. In this method a polymer and a low-solubility drug are dissolved in a solvent and spray-dried to form dispersions in which the drug is mostly present in an amorphous form rather than in a crystalline form. The product form is believed to be a solid solution. That is, both the drug and the polymer are present in a single, undifferentiated phase. Solid solutions are believed to form only at particular component ratios and there is therefore little formulation flexibility with these systems.

### Brief Description Of The Invention

We have now determined that an improvement to the known process is obtained if single or miscible solvents which comprise a single phase are used instead of mixtures of immiscible aqueous/non-aqueous solvents that can form an emulsion.

Accordingly the present invention provides a process for making contra-soluble nano-dispersions of at most sparingly-soluble materials in a soluble carrier material comprising the steps of:
(i) providing a single phase mixture of:
   a) a solvent or a mixture of miscible solvents,
   b) at least one carrier material soluble in solvent (a), said carrier material being also contra-soluble to payload material (c) and solid at ambient temperature,
   c) at least one payload material which is soluble in solvent (a), and,
(ii) drying the mixture to remove solvent (a) and thereby obtain the carrier material (b) in solid form with payload (c) dispersed therein as nanoparticles.

In the context of the present invention, "insoluble" as applied to payload means that its solubility in the target environment is less than 10g/L at ambient temperature. For water-insoluble payload materials this would be a solubility of less than 10g/L in water. In the context of the present invention nanoparticles are particles which are smaller than one micron and larger than 1 nm.

Payload materials preferably have a normal solubility (from a solid or powder form) at ambient temperature (20 Celsius) in their target environment of less than 5g/L preferably of less than 1g/L, especially preferably less than 120mg/L, even more preferably less than 15mg/L and most preferably less than 5mg/L.

In the compositions obtained by process of the present invention the carrier material (b) and the payload (c) are not present in the same phase after drying. The payload is believed to be present as a nanoparticle phase dispersed through a continuous phase of the carrier material.

The preferred method of particle sizing for the dispersed products of the present invention employs a dynamic light scattering instrument ("Nano S", manufactured by Malvern Instruments UK). Specifically, the Malvern Instruments Nano S uses a red (633nm) 4mW Helium-Neon laser to illuminate a standard optical quality UV curvette containing a suspension of material. The particle sizes quoted in this application are those obtained with that apparatus using the standard protocol. Particle sizes in solid products are the particle sizes inferred from the measurement of the particle size obtained by solution of the solid form and subsequent measurement of the particle size.

In the present application the term 'ambient temperature' means 20 degrees Celsius and all percentages are percentages by weight unless otherwise specified.

By using the process as described herein, and an appropriate choice of carrier, it is possible to form a solid matrix that is soluble in a particular aqueous or non-aqueous solvent and which releases the payload (which is insoluble in the particular solvent dissolving the matrix) to form a liquid nano-dispersion of the payload material in the solvent.

In this specification "drying' means the removal of such aqueous and non-aqueous solvents as are present. Typically, the drying process is conducted above ambient temperature. Most preferably, the drying process is a spray-drying process. Less preferable alternatives to spray-drying include freeze-drying which is conducted below ambient temperature. The feedstock to the dryer is essentially free of solids (when at ambient temperature). Where solids are present in the dryer feedstock these should not comprise more than 10%, preferably not more that 5% of the feedstock.

In one alternative form of the invention a non-aqueous solvent is employed in which both the payload and the carrier are soluble. This solvent can comprise a single solvent species or a mixture of solvent species. Preferred solvents are polar, protic or aprotic solvents. Generally preferred solvents have a dipole moment greater than 1 and a dielectric constant greater than 4.5.

Particularly preferred solvents are selected from the group consisting of haloforms (preferably dichloromethane, chloroform), lower (C1-C10) alcohols (preferably methanol, ethanol, isopropanol, isobutanol), organic acids (preferably formic acid, acetic acid), amides (preferably formamide, N,N-dimethylformamide), nitriles (preferably aceto-nitrile), esters (preferably ethyl acetate) aldehydes and ketones (preferably methyl ethyl ketone, acetone), and other water miscible species comprising a hetroatom bond with a suitably large dipole (preferably tetrahydrofuran, dialkylsulphoxide).

In an alternative form of the invention a mixture of miscible solvents are used in which mixture both the payload and the matrix are soluble, and wherein at least one of the solvents is an aqueous solvent and at least another solvent is a non-aqueous solvent. Preferably, the non-aqueous solvent is selected from one or more of the polar protic or polar aprotic solvents listed above.

It is also possible to use a solvent which comprises only an aqueous phase provided it can dissolve both an oil-insoluble payload material and a carrier which is also oil-soluble. This method is used for the production of materials which comprise a nano-dispersion of a water-soluble material in an oil-soluble carrier.

Solvent systems employed in the present invention are not limited to binary mixtures, but can include three or more components. Additional water-immiscible solvents can be present provided that they are miscible in the solvent mixture as a whole. These additional water-immiscible solvents need not fulfill the conditions as regards either dipole moment and/or dielectric constant as given above and include, for example, linear hydrocarbons (preferably hexane), cyclic hydrocarbons (preferably cyclohexane), halocarbons (preferably carbon tetrachloride, methylene chloride) and ethers (preferably diethyl ether).
While the 'carrier' and the 'payload' material are both soluble in a single phase (which can be a single solvent or a mixture of solvents) the use of an emulsion in the drying process is not altogether excluded as other materials may be present which are not miscible provided that there exists at least one single phase which comprises both the carrier and the payload.

Where a mixture of solvents is used it is not necessary that the solvents are mixed before the carrier and the payload material are dissolved therein. It is possible to dissolve the carrier and the payload in different solvents which are then mixed prior to the drying step.

Preferably the solvents present are removed simultaneously, rather than sequentially, in a single drying step.

Ideally, where an insoluble payload is being delivered into an aqueous environment, the carrier material is rapidly soluble in water as well as in the solvent or solvent mixtures used to form the feed for the drying step. Thus, when the solid product is placed in an aqueous environment, the carrier will quickly dissolve and release the payload material in nano-disperse form.

Where the carrier will deliver a material that is water-soluble into a non-aqueous environment, the carrier should preferably be rapidly soluble in that environment. In the present specification the term 'oil' is used to indicate a non-aqueous environment. Thus, when the solid product is placed in oil, the carrier will quickly dissolve and release the payload material in a nano-disperse form.

As noted above, typical particle sizes for the dispersed form of the payload material after delivery may be determined by use, for example, of a Malvern™ 'Nano-S' apparatus. Results generally fall into the range from 500-2nm (expressed as a diameter) and this particle size range is preferred, with 300-4nm being particularly preferred. For comparative purposes, the range is analogous to the size of a virus particle (which typically range from 450-20 nm). The size distribution may show more than one peak. Thus, as noted above, the "solutions" obtained are not true solutions comprising a material dispersed on a molecular scale but are nano-scale dispersions in which the `soluble' material retains some organized structure but on such a small scale that it has many of the properties of a true solution. For example, dispersing a water-insoluble bleach from products of the present invention results in the bleach being more finely dispersed and reduces the spot damage seen when larger water-insoluble particles of the bleach contact a fabric.

It is believed that, especially in the case of non-polymers, many of the particles of "payload' materials present both in the dry (solvent free) form and the re-dispersed product are substantially crystalline materials. This can be seen by X-ray powder diffraction, which has shown diffraction patterns in the dried material characteristic of a crystalline structure. Preferably more that 50% of the payload material is in a crystalline form.

Differential scanning calorimetry of the dried material has also shown melting behavior which is indicative of the presence of organized structures. From these results, it is believed that the compositions of the invention are not solid solutions, but comprise a nano-scale, phase-separated mixture. Moreover, the compositions of the present invention can be varied as regards the ratios of the carrier and the payload, again indicating that they are not solid solutions which are generally only formed at specific component ratios.

### Detailed Description

The invention will be further described below with reference to certain preferred features. As noted above, the invention uses a carrier and a payload which are "contra-soluble" in water and a non-aqueous solvent. Within the generality of the present invention are four specific sub-groups of embodiments. These can be briefly described as follows:

### Type I:

A process for preparing a form of a water-insoluble material which is dispersible in water using a mixed aqueous/non-aqueous solvent system,

### Type II:

A process for preparing a form of a water-insoluble material which is dispersible in water using an anhydrous solvent system,

### Type III:

A process for preparing a form of an oil-insoluble material which is dispersible in oil using a mixed aqueous/non-aqueous solvent system, and,

### Type IV:

A process for preparing a form of an oil insoluble material which is dispersible in oil using a wholly aqueous solvent system.

The types of process I to IV will each be described in further detail below. Types I and II are the preferred processes. It will be noted that in both I and II water soluble carrier materials and water insoluble payload materials are used. Preferred payload and carrier materials for processes of types I and II are described if further detail below.

### Preferred water-insoluble 'payload materials':

Some specific examples of water-insoluble 'payload' materials are given below. These are given as examples only and are not intended to limit the applicability of the present invention. Those skilled in the art will however realize that the materials of the present invention will have utility in other areas not specifically exemplified herein.

Water insoluble (hydrophobic) materials that are released from the products obtained by the process of the present invention at the time of use and can be utilized in the process of the present invention preferably include:-
- antimicrobial agents: for example: Triclosan™, climbazole, octapyrox, ketoconazole, propiconazole, phthalimo-peroxyhexanoic acid (PAP), quaternary ammonium compounds;
- antidandruff agents: for example: zinc pyrithione;
- skin lightening agents, for example 4-ethylresorcinol;
- fluorescing agents: for example: 2,5-bis(2-benzoxazolyl) thiophene for use on fabrics (such as cotton, nylon, polycotton or polyester) in laundry products;
- skin conditioning agents: for example: cholesterol;
- antifoaming agents: for example: isoparrafin
- hair conditioning agents: for example: quaternary ammonium compounds, protein hydrolysates, peptides, ceramides and hydrophobic conditioning oils for example hydrocarbon oils such as paraffin oils and/or mineral oils, fatty esters such as mono-, di-, and triglycerides, silicone oils such as polydimethylsiloxanes (e.g. dimethicone) and mixtures thereof;
- fabric conditioning agents: for example: quaternary ammonium compounds having 1 to 3, preferably 2 optionally substituted (C8-C24) alk(en)yl chains attached to the nitrogen atom by one or more ester groups; hydrophobic monoparticles such as a sucrose polyester for example sucrose tetra-tallowate; silicones for example polydimethylsiloxane;
- thickening agents: for example: hydrophobically modified cellulose ethers such as modified hydroxyethyl-celluloses;
- dyes and colouring agents: for example: dyes intended to change the colour of fabrics, fibres, skin or hair;
- UV protecting agents: such as sunscreens for example:
   octyl methoxycinnamate (Parsol MCX), butyl methoxydibenzoylmethane (Parsol 1789) and
   benzophenone-3 (Uvinul M-40), ferulic acid;
- bleach or bleach precursors: for example: 6-N-phthalimidoperoxyhexanoic acid (PAP) or photobleaching compounds.
- Antioxidants: for example: hydrophobic vitamins such as vitamin E, retinol, antioxiants based on hydroxytoluene such as Irganox™ or commercially available antioxidants such as the Trollox™ series.
- Insecticides (for example lamda-cyhalothin), pesticides, herbicides and other agrochemicals, in particular, those that are stored as solid compositions before use but which are made up into liquid for spraying (or other application) onto animals or crops (for example lamda-cyhalothin),
- perfumes or flavourings or precursors thereto;
- pharmaceutically or veterinary active materials;
- Vitamins/nutraceuticals

Whether a type I or a type II process is used will depend on whether the water-insoluble payload material is soluble in a wholly non-aqueous solvent or a water-containing mixture of solvents. If the payload material is only soluble in a mixture containing water then the type I process (mixed aqueous/non-aqueous solvents) must be used.

Suitable carrier materials include the preferred water-soluble polymers, preferred water-soluble surfactants and preferred water-soluble inorganic materials as described in further detail below. These lists are not exhaustive as other water-soluble materials, for example citric acid, can be used.

Carrier materials for processes of types I and II are water soluble as well as being soluble in at least one of the aqueous/non-aqueous solvent system of type I processes or the anhydrous solvent system of type II processes. As these materials are all water-soluble they will generally be soluble in the solvent combinations of the type I process.

### Preferred polymeric carrier materials:

Examples of suitable water-soluble polymeric carrier materials include:
   (a) natural polymers (for example naturally occurring gums such as guar gum, alginate locust bean gum or a polysaccharide such as dextran;
   (b) cellulose derivatives for example xanthan gum, xyloglucan, cellulose acetate, methylcellulose, methylethylcellulose, hydroxy-ethylcellulose, hydroxyethylmethyl-cellulose, hydroxy-propylcellulose, hydroxy-propylmethylcellulose, hydroxy-propylbutylcellulose, ethylhydroxy-ethylcellulose, carboxy-methylcellulose and its salts (eg the sodium salt - SCMC), or carboxy-methylhydroxyethylcellulose and its salts (for example the sodium salt);
   (c) homopolymers of or copolymers prepared from two or more monomers selected from: vinyl alcohol, acrylic acid, methacrylic acid, acrylamide, methacrylamide, acrylamide methylpropane sulphonates, aminoalkylacrylates, aminoalkyl-methacrylates, hydroxyethylacrylate, hydroxyethylmethylacrylate, vinyl pyrrolidone, vinyl imidazole, vinyl amines, vinyl pyridine, ethyleneglycol and other alkylene glycols, ethylene oxide and other alkylene oxides, ethyleneimine, styrenesulphonates, ethyleneglycolacrylates and ethyleneglycol methacrylate
   (d) cyclodextrins, for example beta-cyclodextrin
   (e) mixtures thereof

When the polymeric material is a copolymer it may be a statistical copolymer (heretofore also known as a random copolymer), a block copolymer, a graft copolymer or a hyperbranched copolymer. Comonomers other than those listed above may also be included in addition to those listed if their presence does not destroy the water soluble or water dispersible nature of the resulting polymeric material.

Examples of suitable and preferred homopolymers include poly-vinylalcohol, poly-acrylic acid, poly-methacrylic acid, poly-acrylamides (such as poly-N-isopropylacrylamide), polymethacrylamide; poly-acrylamines, poly-methyl-acrylamines, (such as polydimethylaminoethylmethacrylate and poly-N-morpholinoethylmethacrylate), polyvinylpyrrolidone, polystyrenesulphonate, polyvinylimidazole, polyvinylpyridine, poly-2-ethyl-oxazoline poly-ethyleneimine and ethoxylated derivatives thereof.

Polyethylene glycol (PEG), Polyvinyl alcohol (PVA), polyvinylpyrrolidone (PVP), hydroxypropyl celluose and/or hydroxypropyl methyl-cellulose (HPMC) are particularly preferred polymer carrier materials.

### Preferred surfactant carrier materials:

Where the carrier material is a surfactant, the surfactant may be non-ionic, anionic, cationic, amphoteric or zwitterionic.

Examples of suitable non-ionic surfactants include ethoxylated triglycerides; fatty alcohol ethoxylates; alkylphenol ethoxylates; fatty acid ethoxylates; fatty amide ethoxylates; fatty amine ethoxylates; sorbitan alkanoates; ethylated sorbitan alkanoates; alkyl ethoxylates; Pluronics™; alkyl polyglucosides; stearol ethoxylates; alkyl polyglycosides.

Examples of suitable anionic surfactants include alkylether sulfates; alkylether carboxylates; alkylbenzene sulfonates; alkylether phosphates; dialkyl sulfosuccinates; alkyl sulfonates; soaps; alkyl sulfates; alkyl carboxylates; alkyl phosphates; paraffin sulfonates; secondary n-alkane sulfonates; alpha-olefin sulfonates; isethionate sulfonates.

Examples of suitable cationic surfactants include fatty amine salts; fatty diamine salts; quaternary ammonium compounds; phosphonium surfactants; sulfonium surfactants; sulfonxonium surfactants.

Examples of suitable zwitterionic surfactants include N-alkyl derivatives of amino acids (such as glycine, betaine, aminopropionic acid); imidazoline surfactants; amine oxides; amidobetaines.

Mixtures of surfactants may be used. In such mixtures there may be individual components which are liquid, provided that the carrier material overall, is a solid.

Alkoxylated nonionic's (especially the PEG/PPG eg Pluronic™ materials and/or the PEG/alcohol nonionics and/or aromatic nonionics, including phenol-ethoxylates (especially Triton™ materials), alkyl sulphonates (especially SDS), ether-sulphates (including SLES), ester surfactants (preferably sorbitan esters of the Span™ and Tween™ types) and cationics (especially cetyltrimethylammonium bromide - CTAB) are particularly preferred as surfactant carrier materials.

### Preferred inorganic carrier materials:

In the type I and type II process the carrier material can also be a water-soluble inorganic material which is neither a surfactant not a polymer. Simple organic salts have been found suitable, particularly in admixture with polymeric and/or surfactant carrier materials as described above. Suitable salts include carbonate, bicarbonates, halides, sulphates, nitrates and acetates, particularly soluble salts of sodium, potassium and magnesium. Preferred materials include, sodium carbonate, sodium bicarbonate and sodium sulphate. These materials have the advantage that they are cheap and physiologically acceptable. They are also relatively inert as well as compatible with many materials found in household and pharmaceutical products.

### Preferred combinations of carrier materials:

Mixtures of carrier materials are advantageous.

For the type I and type II process, preferred mixtures include combinations of inorganic salts and surfactants and, in particular, mixtures of polymers and surfactants. Particularly preferred mixtures include combinations of surfactants and polymers which include at least one of:
a) Polyethylene glycol (PEG), polyvinylpyrrolidone (PVP), poly(2-ethyl-2-oxazaline), polyvinyl alcohol (PVA) hydroxypropyl cellulose and hydroxypropyl-methyl cellulose (HPMC), alginates and mixtures thereof,
   and, at least one of;
b) alkoxylated nonionic's (especially the PEG/PPG Pluronic™ materials), phenol-ethoxylates (especially TRITON™ materials), alkyl sulphonates (especially SDS), ether-sulphates (including SLES), ester surfactants (preferably sorbitan esters of the Span™ and Tween™ types), cationics (especially cetyltrimethylammonium bromide - CTAB), and mixtures thereof

In preferred forms of the invention the level of surfactant carrier is such that at least 50% of the total carrier is surfactant. Mixtures having a majority of surfactant present over the other carriers exhibit enhanced payload effects.

### Preferred non-aqueous solvents:

Both processes of types I and II use a non-aqueous solvent (or a mixture of said solvents).

Particularly preferred solvents are selected from the group consisting of haloforms (preferably di-chloromethane, chloroform), lower (C1-C10) alcohols (preferably methanol, ethanol, isopropanol, isobutanol), organic acids (preferably formic acid, acetic acid), amides (preferably formamide, N,N-dimethylformamide), nitriles (preferably aceto-nitrile), esters (preferably ethyl acetate) aldehydes and ketones (preferably methyl ethyl ketone, acetone), and other water miscible species comprising hetroatom bond with a suitably large dipole (preferably tetrahydrofuran, dialkylsulphoxide). Mixtures of the aforementioned may also be employed.

Preferred solvents include dichloromethane, chloroform, ethanol, acetone and dimethyl sulphoxide.

Preferred non-aqueous solvents have a boiling point of less than 150 Celsius and, more preferably, have a boiling point of less than 100 Celsius, so as to facilitate drying, particularly spray-drying under practical conditions and without use of specialised equipment. Preferably they are non-flammable, or have a flash point above the temperatures encountered in the method of the invention.

Process of types I and II are described in further detail below:

### Type I: nano-dispersible forms of a water-insoluble material prepared using a mixed solvent system

This aspect of the invention provides a process for making a nano-dispersion of a water-in soluble material in a water-soluble carrier material comprising the steps of:
(i) providing a mixture of:
   a) an aqueous solvent for a carrier material;
   b) at least one water-soluble carrier material soluble in the mixture of aqueous solvent (a) and second solvent (c), said carrier material being solid at ambient temperature,
   c) at least one non-aqueous second solvent said second solvent being miscible with aqueous solvent (a) to form a mixed solvent capable of dissolving carrier material (b) and payload (d), and;
   d) a payload material which is dispersible or soluble in the mixture of aqueous solvent (a) and non-aqueous solvent (c) but not in aqueous solvent (a) alone, and;
(ii) drying the mixture at above ambient temperature to remove solvents (a) and (c) and thereby obtain the carrier material (b) in a solid form with payload (d) dispersed therein in a nanoparticulate form, wherein the product of the drying step is water dispersible to give an aqueous solution of (b) and an aqueous nano dispersion of (d).

### Preferred aqueous solvents:

The preferred aqueous solvent is water. Electrolyte solutions can also be used as the aqueous solvent.

Particular examples of water miscible non-aqueous solvents, payload materials soluble in the solvent and water-soluble carrier materials also soluble in a mixture of the solvent and an aqueous solution are provided in Table 1 below.

**Table 1**

| **Water-miscible solvents to make water soluble products** | | |
|---|---|---|
| **Non-limiting examples of water-insoluble payload materials** | **Water-miscible non-aqueous solvents for the payload material** | **Water-soluble carriers soluble in mixture of solvent and aqueous solution** |
| Triclosan | Ethanol Acetone Isopropyl alcohol | SDS |
| Sudan Red 7B | Acetone | SDS |

### Type II: nano-dispersible (in water) forms of a water-insoluble material using a single solvent system

This aspect of the invention provides a process for making water-soluble nano-dispersions of at most sparingly water-soluble materials in a water-soluble carrier material comprising the steps of:
(i) providing a mixture of:
   a) a non-aqueous solvent or a mixture of miscible non-aqueous solvents,
   b) at least one carrier material soluble in non-aqueous solvent (a), said carrier material being also soluble in water and solid at ambient temperature,
   c) at least one water-insoluble payload material which is soluble in non-aqueous solvent (a), and,
(ii) drying the mixture above ambient temperature to remove solvent (a) and thereby obtain the carrier material (b) in solid form with payload material (c) dispersed therein in a nanoparticulate form, wherein the product of the drying step is water dispersible to give an aqueous solution of (b) and an aqueous nano dispersion of (c).

Suitable carrier materials include the preferred water-soluble polymers and preferred water-soluble surfactants as mentioned above which are also soluble in the anhydrous non-aqueous solvent (or solvent mixture)

Particularly preferred carrier materials for use in process type II are:
a) polyethylene glycol (PEG), polyvinylpyrrolidone (PVP), polyvinyl alcohol (PVA) hydroxypropyl cellulose (HPC) and hydroxypropyl-methyl cellulose (HPMC) preferably in combination with at least one of;
b) alkoxylated non-ionic's (especially the PEG/PPG Pluronic™ materials), phenol-ethoxylates (especially TRITON™ materials), alkyl sulphonates (especially SDS), ether-sulphates (including SLES), ester surfactants (preferably sorbitan esters of the Span™ and Tween™ types) and cationics (especially cetyltrimethylammonium bromide - CTAB)

Particularly preferred solvents are haloforms for use in process type II (preferably di-chloromethane, chloroform), lower (C1-C10) alcohols (preferably methanol, ethanol, isopropanol, isobutanol), organic acids (preferably formic acid, acetic acid), amides (preferably formamide, N,N-dimethylformamide), nitriles (preferably aceto-nitrile), esters (preferably ethyl acetate) aldehydes and ketones (preferably methyl ethyl ketone, acetone), and species comprising a hetroatom bond with a suitably large dipole (preferably tetrahydrofuran, dialkylsulphoxide). Mixtures of the aforementioned may also be employed.

The presence of some low level of water in the non-aqueous solvent is not excluded, as a cheaper technical grade of the solvent may be used rather than a more expensive analytical grade. Preferably the level of water is below 1%.

Particular examples of non-aqueous solvents, payload materials soluble in the solvent and carrier materials also soluble in the solvent are provided in Table 2 below.

**Table 2**

| **Single phase solvents/carriers to make water "soluble"products** | | |
|---|---|---|
| **Non-limiting examples of water-insoluble payload materials** | **Water-immiscible non-aqueous solvents** | **Water-soluble carriers soluble in solvent** |
| Chlorothalonil (fungicide) | Chloroform | PVP PEG-PPG |
| Amphomer Resin (Resin-AM) | Ethanol | Hydroxy propyl cellulose |
| Azoxystrobin | dichloromethane | PVP Phenol-ethoxylate |
| Azoxystrobin | dichloromethane | PVP PEG-PPG |
| Divanillin | DMSO | PVP HPC |
| Polycaprolactone | DCM | PVP PEG-PPG |
| 1-Napthylamine | DCM | PVP PEG-PPG |
| Lambda Cyhalothrin | DCM | PPV PEG-PPG |
| Homoeriodictol | DMSO | PVP HPC |
| Sudan Red Oil Red O(dyes) | Chloroform Dichloromethane | PEG-PPG PVP |

These combinations of materials give a product which is dispersible in water to form a nano-dispersion of the water-insoluble payload material.

Some materials exhibit a low solubility in both aqueous and non-aqueous solvents (examples are Homoeriodictyol and divanillin), both of which are insoluble in water, ethanol and poly-propylene glycol. However these both materials are soluble in DMSO. By selection of a carrier which is also soluble in both DMSO and one of water, ethanol and PPG (for example PVP/HPC) it is possible to obtain a nano-disperse form of either payload material in the carrier, which can be "dissolved" in water, ethanol or PPG.

Spray drying, the most preferred method of drying, is well known to those versed in the art. In the case of the present invention some care must be taken due to the presence of a volatile non-aqueous solvent in the feedstock being dried. In order to reduce the risk of explosion when a flammable solvent is being used, an inert gas, for example nitrogen, can be employed as the drying medium in a so-called closed spray-drying system. The solvent can be recovered and reused.

We have found that the 'Buchi' B-290 type laboratory spray drying apparatus is suitable.

It is preferable that the drying temperature should be at or above 100 Celsius, preferably above 120 Celsius and most preferably above 140 Celsius. Elevated drying temperatures have been found to give smaller particles in the re-dissolved nano-disperse material.

In addition to the non-aqueous solvent an optional co-surfactant may be employed in the composition prior to the drying step. We have determined that the addition of a relatively small quantity of a volatile cosurfactant reduced the particle diameter of the material produced. This can have a significant impact on particle volume. For example, reduction from 297nm to 252nm corresponds to a particle size reduction of approximately 40%. Thus, the addition of a small quantity of co-surfactant offers a simple and inexpensive method for reducing the particle size of materials according to the present invention without changing the final product formulation.

Preferred co-surfactants are short chain alcohols or amine with a boiling point of <220°C.

Preferred co-surfactants are linear alcohols. Preferred co-surfactants are primary alcohols and amines. Particularly preferred co-surfactants are selected from the group consisting of the 3-6 carbon alcohols. Suitable alcohol co-surfactants include n-propanol, n-butanol, n-pentanol, n-hexanol, hexylamine and mixtures thereof.

Preferably the co-surfactant is present in a quantity (by volume) less than the solvent preferably the volume ratio between the solvent and the co-surfactant falls in the range 100:40 to 100:2, more preferably 100:30 to 100:5.

### Type III and IV processes:

In addition to the processes in which the carrier is water-soluble and the payload is water-insoluble, it is also possible to use an oil-soluble carrier to deliver a contra-soluble (i.e. oil-insoluble) payload into a non-aqueous environment.

As with the processes of types I and II for making water soluble/dispersible products, the process used to obtain an oil soluble product may use a mixed aqueous/non-aqueous solvent for the water soluble payload material (as in type III). In the alternative it may simply use water (as in type IV) provided that the oil soluble carrier is also soluble in water. These processes of types III and IV are described in further detail below.

### Type III: nano-dispersible (in oil) forms of an oil-insoluble material using a mixed solvent system

This aspect of the invention provides a method of making a nano-dispersion of oil-insoluble material in an oil-soluble carrier material comprising the steps of:
(i) providing a mixture of:
   a) a non-aqueous solvent for a carrier material,
   b) an oil-soluble carrier material which is also soluble in the mixture of non-aqueous solvent (a) and aqueous solvent (c), said carrier material being solid at ambient temperature,
   c) a second aqueous solvent for a payload material, said aqueous solvent being miscible with non-aqueous solvent (a), and,
   d) a payload material which is soluble in the mixture of solvents (a) and (c) but not in (a) alone, and,
(ii) drying the mixture above ambient temperature to simultaneously remove solvents (a) and (c) and thereby obtain the carrier material (b) in a solid form with the payload (d) dispersed therein in a nanoparticulate form, wherein the product of the drying step is oil dispersible to give an oil solution of (b) and a nano dispersion of (c).

Suitable aqueous solvents include water and electrolyte solutions.

Suitable carrier materials (referred to herein as 'water soluble carrier materials') include preferred water-soluble polymers, preferred water-soluble surfactants and preferred water-soluble inorganic materials. Of the preferred carrier materials mentioned above, the surfactants and the polymers are the preferred carriers as these have adequate solubility in water and an adequate solubility in non-aqueous solvents.

Particularly preferred carriers include poly-ethylene glycol (PEG), poly-vinyl pyrrolidone (PVP), alkoxylated non-ionic's (especially the PEG/PPG Pluronic™ materials), and mixtures thereof.

Particular examples of non-aqueous solvents, payload materials soluble in the aqueous solvent and carrier materials also soluble in the solvent are provided in Table 3 below.

**Table 3**

| **Solvents/Carriers to make "oil soluble" products** | | |
|---|---|---|
| **Non-limiting examples of oil-insoluble payload materials** | **Solvents for the carrier** | **Oil-soluble carriers also soluble in water** |
| Sodium Sulphate | Water | PVP PEG-PPG-PPG |
| Poly vinyl alcohol | Water | PEG-PPG-PEG PVP |

These products are dispersible in non-aqueous solvents ('oils') such as chloroform and ethanol to form a nano-dispersion of the payload in the non-aqueous solvent. By way of example, if the process of type III is used to disperse sodium sulphate in a carrier which is a mixture PVP and PEG/PPG, then the resulting product can be dissolved in an oil (such as chloroform, in which sodium sulphate is insoluble) to obtain a nano-dispersion of sodium sulphate in a solution of the carrier in chloroform.

### Type IV: oil-soluble forms of an oil-insoluble material using a single solvent system

This aspect of the invention provides a method of making oil-soluble nano-dispersions of at most sparingly oil-soluble materials in a oil-soluble carrier material preferably comprising the steps of:
(i) providing a mixture of:
   a) an aqueous solvent,
   b) a carrier material soluble in aqueous solvent (a), said carrier material being also soluble in oil and solid at ambient temperature,
   c) an oil-insoluble payload material which is soluble in aqueous solvent (a), and,
(ii) drying the mixture above ambient temperature to remove (a) and thereby obtain the material (b) in solid form with (c) dispersed therein in a nanoparticulate form, wherein the product of the drying step is oil-dispersible to give an oil solution of (b) and a nano dispersion of (c).

Particular examples of non-aqueous solvents, payload materials soluble in the solvent and carrier materials also soluble in the solvent are provided in Table 4 below.

**Table 4**

| **Single phase solvents to make oil soluble products** | | |
|---|---|---|
| **Non-limiting examples of oil-insoluble payload materials** | **Aqueous solvents** | **Oil-soluble carriers soluble in solvent** |
| Poly vinyl alcohol | Water | Hydroxypropyl cellulose PVP |

These materials enable the formation of a material from which the oil-insoluble payload material is dispersible in an 'oil' (such as propylene glycol) to form a nano-dispersion.

In order that the present invention may be further understood it is further explained below with reference to specific examples and embodiments.

### Examples:

All the particle size measurements are identical; where 1mg spray dried powder was dispersed into 1 ml distilled water. The dispersion was vortex mixed before DLS measurement. All the particle size data were quoted as Z-average.

### Example 1: Nano-dispersions of Chlorothalonil (a water-insoluble fungicide) from a single phase chloroform solution stabilised with PVP and PEG-PPG-PEG

A solution was prepared of the following:

### Composition

- Chlorothalonil: 0.2g (10wt.%)
- PEG-PPG-PEG: 0.4g (20wt.%)
- PVP (90kDa): 1.4g (70wt.%)
- Chloroform: 40ml

At these concentrations, the solid components were readily soluble in the chloroform at the measured room temperature (21.5°C).

The solution was spray dried using a Buchi B-290™ bench top spray-dryer, operated in a negative pressure mode. Air drawn from the lab was used as the drying medium and the operating conditions were as follows:
- Pump rate: 10% (3.6ml/min)
- Inlet temperature: 105°C
- Aspiration: 100%
- N₂ flow (atomisation): Max (approx. 55L/hr)

A dry white powder was obtained. This material was redispersed in demineralised water at a concentration of 10mg/ml (1.0wt%, 0.1wt% chlorothalonil). This produced an opaque white dispersion. At this concentration, the material was relatively slow to disperse (approx. 5 minutes).

The resulting solution had the following properties:
- Viscosity: 1.95cP
- Particle size: 437nm (diameter)
- Standard deviation: ± 25.5nm
- PdI: 0.385

### Example 2: Nano-dispersion of Chlorothalonil (a fungicide) from a single phase chloroform solution stabilised with PVP and PEG-PPG-PEG

### Composition

- Chlorothalonil: 0.2g (10wt.%)
- PEG-PPG-PEG: 0.4g (20wt.%)
- PVP (55kDa): 1.4g (70wt.%)
- Chloroform: 40ml

At these concentrations, the solid components are readily soluble in chloroform at room temperature (21.5°C).

### Drying

The solution was spray dried using a Buchi B-290 bench top spray dryer, operated in a negative pressure mode. Air drawn from the lab was used as the drying medium.
- Pump rate: 15% (5.4ml/min)
- Inlet temperature: 90°C
- Aspiration: 100%
- N₂ flow (atomisation): Max (approx. 55L/hr)

### Product

A dry white powder was obtained. This material was redispersed in demineralised water at a concentration of 1mg/ml (0.1wt%, 0.01wt% chlorothalonil). This produced an opaque white dispersion. At this concentration, the material was considerably quicker (than example 1) to disperse (less than 30 seconds).
- Viscosity: 1.0cP
- Particle size: **452nm** (diameter)
- Standard deviation: ± 5.72nm
- PdI: 0.181

### Example 3: Nano-dispersion of Chlorothalonil (fungicide) from a single phase chloroform solution stabilised with PVP and PEG-PPG-PEG

### Composition

- Chlorothalonil: 0.05g (10wt.%)
- PEG-PPG-PEG: 0.1g (20wt.%)
- PVP (55kDa): 0.35g (70wt.%)
- Chloroform: 30ml

At these concentrations, the solid components are readily soluble in chloroform at room temperature (21.5°C).

### Drying

The solution was spray dried using a Buchi B-290 bench top spray dryer, operated in a negative pressure mode. Air drawn from the lab was used as the drying medium.
- Pump rate: 15% (5.4ml/min)
- Inlet temperature: 90°C
- Aspiration: 100%
- N₂ flow (atomisation): Max (approx. 55L/hr)

### Product

A dry white powder was obtained. This material was redispersed in demineralised water at a concentration of 1mg/ml (0.1wt%, 0.01wt% chlorothalonil). This produced an opaque white dispersion. At this concentration, the material dispersed at a similar rate to example 2 (less than 30 seconds).
- Viscosity: 0.93cP
- Particle size: **402nm** (diameter)
- Standard deviation: ±15.1nm
- PdI: 0.228

### Example 4-8: Nano-dispersion of Sudan Red 7B (water insoluble dye.

### Example 4

0.50 g Sudan red 7B (Aldrich) and 4.50 g Poly(ethylene glycol)-*block*-poly(propylene glycol)-*block*-poly(ethylene glycol) (Mw 8,400, Aldrich) were dissolved into 50 ml Chloroform. The solution was then spray dried at 90°C.

The dry powder was then dispersed into distilled water and the nanoparticle size was measured with Malvern Nano-S.

### Example 5

1.50 g Sudan red 7B (Aldrich) and 3.50 g Poly(ethylene glycol)-*block*-poly(propylene glycol)-*block*-poly(ethylene glycol) (Mw 8,400, Aldrich) were dissolved into 50 ml Dichloromethane (DCM). The solution was then spray dried at 70°C.

The dry powder was then dispersed into distilled water and the nanoparticle size was measured with Malvern Nano-S.

### Example 6

0.50 g Sudan red 7B, 1.00 g Poly(ethylene glycol)-*block-*poly(propylene glycol)-*block*-poly(ethylene glycol) (Mw 8,400, Aldrich), and 3.50 g Polyvinylpyrrolidone (Mw 55,000, Aldrich) were all dissolved into 50 ml Dichloromethane (DCM). The solution was then spray dried at 90°C.

The dry powder was then dispersed into distilled water and the nanoparticle size was measured with Malvern Nano-S.

### Example 7

0.50 g Sudan red 7B, 1.00 g Poly(ethylene glycol)-*block-*poly(propylene glycol)-*block*-poly(ethylene glycol) (Mw 8,400, Aldrich), and 3.50 g Polyvinylpyrrolidone (Mw 55,000, Aldrich) were all dissolved into 50 ml DCM. The solution was then spray dried at 60 °C.

The dry powder was then dispersed into distilled water and the nanoparticle size was measured with Malvern Nano-S. It was found to have two peaks.

### Example 8

1.50 g Sudan red 7B, 1.00 g Poly(ethylene glycol)-*block-*poly(propylene glycol)-*block*-poly(ethylene glycol) (Mw 8,400, Aldrich), and 2.50 g Polyvinylpyrrolidone (Mw 55,000, Aldrich) were all dissolved into 50 ml DCM. The solution was then spray dried at 90 °C.

The dry powder was then dispersed into distilled water and the nanoparticle size was measured with Malvern Nano-S. (WP-123)

Details concerning examples 4-8 and their results are given in Table 6

**Table 6**

| **Ex.** | **Sudan red 7B, mg/ml** | **Pluronic, mg/ml** | **PVP, mg/ml** | **Solvent, ml** | **Spray dry temp., °C** | **PS, nm** |
|---|---|---|---|---|---|---|
| 4 | 30.0 | 70.0 | - | Chloroform, 50.0 | 70 | 203 |
| 5 | 30.0 | 70.0 | - | DCM, 50.0 | 70 | 290 |
| 6 | 10.0 | 20.0 | 70.0 | DCM, 50.0 | 90 | 66 |
| 7 | 10.0 | 20.0 | 70.0 | DCM, 50.0 | 60 | 172, 50 |
| 8 | 30.0 | 20.0 | 50.0 | DCM, 50.0 | 90 | 110 |
| Spray drying conditions: Aspiration rate: 100%; Pump rate: 3.62 ml/min. | | | | | | |

### Example 9-10: Nano-dispersion of Oil Red O (water insoluble dye)

### Example 9

1.50 g Oil red O (Aldrich), 1.00 g Poly(ethylene glycol)-*block*-poly(propylene glycol)-*block*-poly(ethylene glycol) (Mw 8,400, Aldrich), and 2.50 g Polyvinylpyrrolidone (Mw 55,000, Aldrich) were all dissolved into 50 ml DCM. The solution was then spray dried at 90°C.

The dry powder was then dispersed into distilled water and the nanoparticle size was measured with Malvern Nano-S.

### Example 10

1.50 g Oil red O (Aldrich), 1.00 g Poly(ethylene glycol)-*block*-poly(propylene glycol)-*block*-poly(ethylene glycol) (Mw 8,400, Aldrich), and 2.50 g Polyvinylpyrrolidone (Mw 55,000, Aldrich) were all dissolved into 50 ml DCM. The solution was then spray dried at 70°C.

The dry powder was then dispersed into distilled water and the nanoparticle size was measured with Malvern Nano-S. (WP-134)

Details concerning these experiments and their results are given in Table 7

**Table 7**

| **Ex.** | **Oil Red O, mg/ml** | **Pluronic, mg/ml** | **PVP, mg/ml** | **Solvent, ml** | **Spray dry temp., °C** | **PS, nm** |
|---|---|---|---|---|---|---|
| 9 | 30.0 | 20.0 | 50.0 | DCM, 50.0 | 90 | 192 |
| 10 | 30.0 | 20.0 | 50.0 | DCM, 50.0 | 70 | 281 |
| Spray drying conditions: Aspiration rate: 100%; Pump rate: 3.62 ml/min. | | | | | | |

### Example 11-12: Nano-dispersion of Azoxystrobin (water insoluble fungicide)

### Example 11

1.10 g Azoxystrobin, 2.00 g Brij^{®} 58 (Aldrich), and 6.90 g Polyvinylpyrrolidone (Mw 45,000, Aldrich) were all dissolved into 200 ml DCM. The solution was then spray dried at 70°C.

The dry powder was then dispersed into distilled water giving 1wt% AzB in dispersion and the nanoparticle size was measured with Malvern Nano-S.

### Example 12

5.00 g Azoxystrobin, 10.00 g Poly(ethylene glycol)-*block-*poly(propylene glycol)-*block*-poly(ethylene glycol) (Mw 8,400, Aldrich), and 35.00 g Polyvinylpyrrolidone (Mw 45,000, Aldrich) were all dissolved into 1.0 litre DCM. The solution was then spray dried at 70°C.

The dry powder was then dispersed into distilled water giving 1wt% AzB in dispersion and the nanoparticle size was measured with Malvern Nano-S.

Details concerning these experiments and their results are given in Table 8

**Table 8**

| **Ex.** | **AzB, mg/ml** | **Surfactant, mg/ml** | **PVP, mg/ml** | **Solvent, ml** | **Spray dry temp., °C** | **PS, nm** |
|---|---|---|---|---|---|---|
| 11 | 5.5 | Brij 58, 10.0 | 34.5 | DCM, 200 | 70 | 532 |
| 12 | 5.0 | Pluronic, 10.0 | 35.0 | DCM, 1000 | 70 | 356 |
| Spray drying conditions: Aspiration rate: 100%; Pump rate: 1.80 ml/min. | | | | | | |

### Example 13-14: Nano-dispersion of Poly-caprolactone (biodegradable polyester)

### Example 13

0.50 g Polycaprolactone (PCL, Mw 14,000, Aldrich), 1.00 g Poly(ethylene glycol)-*block*-poly(propylene glycol)-*block-*poly(ethylene glycol) (Mw 8,400, Aldrich), and 3.50 g Polyvinylpyrrolidone (Mw 55,000, Aldrich) were all dissolved into 50 ml DCM. The solution was then spray dried at 90°C. The dry powder was then dispersed into distilled water, ethanol, and water/ethanol mixture (50/50 in volume) and the nanoparticle size was measured with Malvern Nano-S.

### Example 14

1.00 g Polycaprolactone (PCL, Mw 14,000, Aldrich) 2.00 g Poly(ethylene glycol)-*block*-poly(propylene glycol)-*block-*poly(ethylene glycol) (Mw 8,400, Aldrich), and 2.00 g Polyvinylpyrrolidone (Mw 55,000, Aldrich) were all dissolved into 100 ml DCM. The solution was then spray dried at 90°C. The dry powder was then dispersed into distilled water, ethanol, and water/ethanol mixture (50/50 in volume) and the nanoparticle size was measured with Malvern Nano-S.

Details concerning these experiments and their results are given in Table 9

**Table 9**

| **Ex** | **PCL, mg/ml** | **Block copolymerm g/ml** | **PVP, mg/ml** | **DCM, ml** | **Spray dry temp. °C** | **PS, nm** | | |
|---|---|---|---|---|---|---|---|---|
| | | | | | | **In water** | **In Water /EtOH** | **In EtOH** |
| 13 | 10.0 | 20.0 | 70.0 | 50.0 | 90 | 106 | 242 | 389 |
| 14 | 10.0 | 20.0 | 20.0 | 100.0 | 90 | 407 | 678 | 832 |
| Spray drying conditions: Aspiration rate: 100%; Pump rate: 3.62 ml/min. | | | | | | | | |

### Example 15: Nano-dispersion of Amphomer Resin (used in hair sprays)

0.25 g of an Amphomer Resin (28-4910, ex ICI), 0.25 g Klucel^{®} EF (Hydroxypropylcellulose, Mw 80,000, Hercules Inc.), and 4.50 g Polyvinylpyrrolidone (Mw 45,000, Aldrich) were all dissolved into 100 ml Ethanol. The solution was then spray dried at 100 °C. The dry powder was then dispersed into distilled water, and the nanoparticle size was measured with Malvern Nano-S.

Details concerning these experiments and their results are given in Table 10.

**Table 10**

| **Ex.** | **AM, mg/ml** | **Klucel EF, mg/ml** | **PVP, mg/ml** | **EtOH, ml** | **Spray dry temp., °C** | **PS, nm** |
|---|---|---|---|---|---|---|
| 15 | 2.5 | 2.5 | 45.0 | 100 | 100 | 445 |
| Spray drying conditions: Aspiration rate: 100%; Pump rate: 3.62 ml/min. | | | | | | |

### Example 16-18: Nano-dispersion of Triclosan (an antimicrobial) from a single-phase mixture of cosolvents stabilised by SDS.

### Composition

- Triclosan: 2.0g (20wt.%)
- SDS: 8.0g (80wt.%)
- Water miscible solvent: 125ml (50/50^{v}/ᵥ mixture)

Three different water miscible organic solvents were employed (ethanol, acetone and isopropyl alcohol). At these concentrations, the solid components are readily soluble in the cosolvent mixture at room temperature (21.5°C).

### Drying

The solution was spray dried using a Buchi B-290 bench top spray dryer, operated in a negative pressure mode. Air drawn from the lab was used as the drying medium.
- Pump rate: 7% (2.5ml/min)

- Inlet temperature: 120°C
- Aspiration: 100%
- N₂ flow (atomisation): Max (approx. 55L/hr)

### Products

For each different cosolvent system, a dry white powder was obtained. These material was redispersed in demineralised water at a concentration of 1mg/ml (0.1wt%), rapidly producing a crystal clear dispersion that remained stable for more than 12 hours. All the dispersions appeared to produce particles of the similar sizes and distributions.

Details concerning these experiments and their results are given in Table 11.

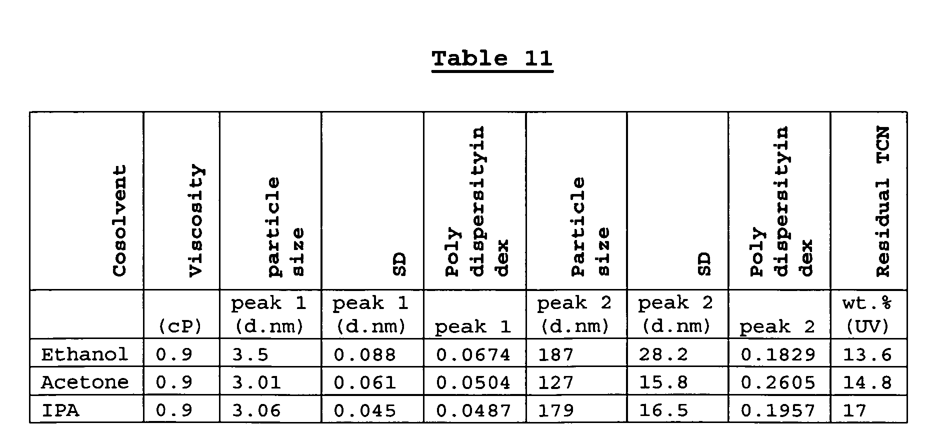

### Example 19: Nano-dispersion of Sudan Red 7B (dye) stabilised with SDS only, spray dried from a mixture of cosolvents.

### Composition

- Sudan Red 7B: 0.2g (4.8wt.%)
- SDS: 4.0g (95.2wt.%)
- Water/Acetone: 200ml(50/50^{v}/ᵥ mixture)

Addition of the cosolvents produced a dark red solution. However, there still appeared to be a very small quantity of undissolved Sudan Red. Therefore, the solution was filtered through a standard Whatman Number 1 filter paper, prior to spray drying.

### Drying

The solution was spray dried using a Buchi B-290 bench top spray dryer, operated in a negative pressure mode. Air drawn from the lab was used as the drying medium.
- Pump rate: 7% (2.5ml/min)
- Inlet temperature: 120°C
- Aspiration: 100%
N₂ flow (atomisation) Max (approx. 55L/hr)

### Products

A dry pink powder was obtained after spray drying. This powder redispersed rapidly at a concentration of 1mg/ml (0.1wt.%) to produce a dark red/purple, transparent solution. Particle sizing of this dispersion revealed two distributions of particle sizes.
- Viscosity:: 0.90cP
- Particle size (peak 1): 39.3nm (diameter)
- Standard deviation (peak 1): ± 5.27nm
- PdI (peak 1): 0.0542
- Particle size (peak 2): 266nm (diameter)
- Standard deviation (peak 2): ± 45.7nm
- PdI (peak 2): 0.0482

### Example 20: Nano-dispersion of Sodium Sulphate (an oil-insoluble salt) which can be dispersed in Chloroform

### Composition

- Sodium Sulphate: 1.0g (10wt.%)
- PEG-PPG-PEG: 2.0g (20wt.%)
- PVP (30kDa): 7.0g (70wt.%)
- Water: 200ml

At these concentrations, the solid components are readily soluble in water at room temperature (21.5°C).

### Drying

The solution was spray dried using a Buchi B-290 bench top spray dryer, operated in a negative pressure mode. Air drawn from the lab was used as the drying medium.
- Pump rate: 10% (3.7ml/min)
- Inlet temperature: 120°C
- Aspiration: 100%
- N₂ flow (atomisation): Max (approx. 55L/hr)

### Product

A dry white powder was obtained. This material was redispersed in chloroform at a concentration of approximately 1mg/ml (0.1wt%, 0.01wt% sodium sulphate). This produced an optically clear/transparent dispersion.
- Viscosity: 0.80cP
- Particle size: **366nm** (diameter) N.B. there are clearly two other smaller particle size distributions at around 10 nm and 60 nm. However, the larger 366 nm peak represents the majority of the particle mass.
- Standard deviation: ± 53.4 nm

### Example 21-23: Nano-dispersion of Polyvinyl alcohol (PVA, an oil-insoluble polymer) in ethanol, chloroform and propylene glycol

### Example 21

0.50 g Polyvinyl alcohol (PVA, Mw 10,000, Aldrich), 1.00 g Poly(ethylene glycol)-*block*-poly(propylene glycol)-*block-*poly(ethylene glycol) (Mw 8,400, Aldrich), and 8.50 g Polyvinylpyrrolidone (Mw 45,000, Aldrich) were all dissolved into 50 ml water and 50 ml ethanol cosolvent. The solution was then spray dried at 90°C. The dry powder was then dispersed into ethanol (EtOH) and the nanoparticle size was measured with Malvern Nano-S. (SP-1)

### Example 22

0.50 g Polyvinyl alcohol (PVA, Mw 10,000, Aldrich), 1.00 g Klucel^{®} EF (Mw 80,000, Hercules Incorporated), and 8.50 g Polyvinylpyrrolidone (Mw 45,000, Aldrich) were all dissolved into 100 ml distilled water. The solution was then spray dried at 150°C. The dry powder was then dispersed into propylene glycol (PPG) and the nanoparticle size was measured with Malvern Nano-S. (SP-2)

Details concerning these experiments and their results are given in Table 12.

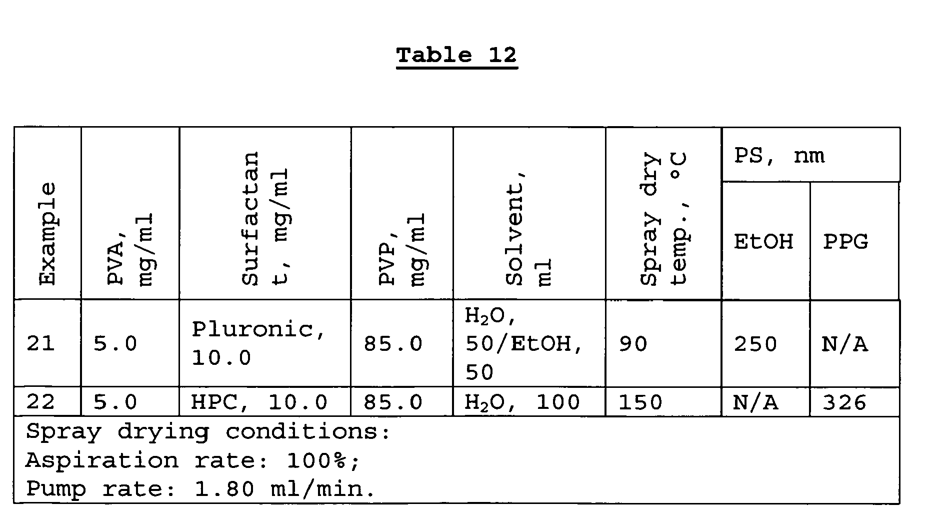

### Example 23-24: Nano-dispersion of Homoeriodictyol (HED) and Divanillin nanoparticles in ethanol or propylene glycol

### Example 23

3.00 g Homoeriodictyol, 2.00 g Klucel^{®} EF (Hydroxypropylcellulose, Mw 80,000, Hercules Incorporated), and 15.00 g Polyvinylpyrrolidone (Mw 45,000, Aldrich) were all dissolved into 600 ml Dimethylsulfoxide (DMSO). The solution was then spray dried at 210 °C. The dry powder was then dispersed into distilled water, ethanol, and propylene glycol, respectively, and the nanoparticle size was measured with Malvern Nano-S. (SY-12)

### Example 24

0.25 g Divanillin, 0.25 g Klucel^{®} EF (Hydroxypropylcellulose, Mw 80,000, Hercules Incorporated), and 4.50 g Polyvinylpyrrolidone (Mw 45,000, Aldrich) were all dissolved into 100 ml Dimethylsulfoxide (DMSO). The solution was then spray dried at 210 °C. The dry powder was then dispersed into distilled water, ethanol, and propylene glycol, respectively, and the nanoparticle size was measured with Malvern Nano-S. (SY-10)

Details concerning these experiments and their results are summarized in Table 13.

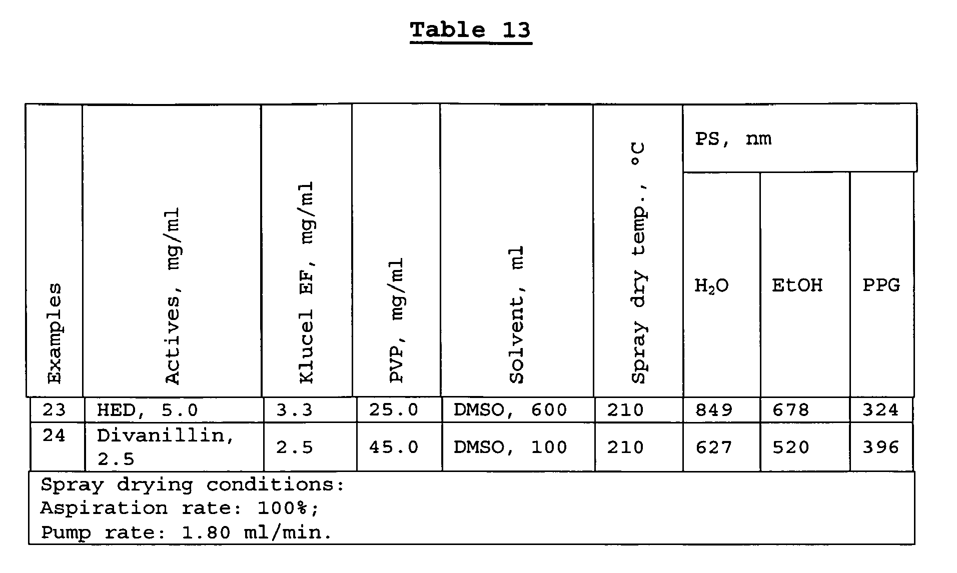

### Example 25: Water dispersible 1-naphthylamine:

70% PVP (30K), 20% Pluronic F68 and 10% 1-naphthylamine dissolved in DCM (10% w/v).

The solution was then spray dried at 50°C with liquid feed rate at 3.02 ml/min. An off-white powder with 10 wt% (theoretical) 1-Naphthylamine was obtained.

The powder was redispersible in water with a particle size Z average measuring 19.8 nm.

### Example 26: Water dispersible climbazole:

70% PVP (30K), 20% Pluronic F68 and 10% climbazole dissolved in Chloroform (2.5% w/v).

The solution was then spray dried at 100°C with liquid feed rate at 3.32 ml/min. A white powder with 10 wt% (theoretical) climbazole was obtained.

The powder was redispersible in water with a particle size Z average measuring 166 nm.

### Example 27: Water dispersible X-Cyhalothrin:

400 mg X-Cyhalothrin (20%), 360 mg Pluronic F68 (18%) and 1240 mg PVP-k30 (62%) were dissolved in 20 ml DCM to produce a pale brown translucent liquid.

This solution was then spray dried at an inlet temperature of 160°C and a pump rate of 3.6 ml/min.

The resulting dry, white/pale brown powder was redispersed in water to produce a clear dispersion at a concentration of 1 mg/ml. The dispersions particle size was measured by DLS (see particle size trace below). Z-average (mass based size) was found to be 94.2 nm.

### Example 28: Tinopal SOP™(three solvent system/surfactant carrier)

18g of cocoPAS were dissolved in 240ml water and then 185ml of ethanol was added. 2g of Tinopal™ SOP was dissolved in 240ml dichloromethane and then 185ml of ethanol was added to the resulting solution.

The two solutions were combined and spray-dried using a Buchi mini B-290 with inlet temperature set at 125°C and a 10% pump rate to give a pale yellow powder.

On dispersion of 1mg of the powder into 1ml of water a nanoparticle size of 219nm (Zave) was obtained.

### Example 29: Vitamin E (single solvent/polymer + surfactant carrier)

0.6g PVP (Mw 30K), 0.3g Tween™ 40 and 0.1g vitamin E were dissolved in 70ml ethanol.

The resulting solution was spray dried using a Buchi mini B-290 system. The Inlet temperature was set at 150°C and the pump rate adjusted to approximately 3.6 ml/min.

The product was recovered as a dry white powder which was redispersed in water to a concentration of 1 mg/ml and the particle size was measured using a Malvern Nano-S and found to be 17 nm (Zave)

### Example 30: Vitamin E acetate (single solvent/polymer + surfactant carrier)

0.6g HPC, 0.3g Tween™ 40 and 0.1g vitamin E acetate were dissolved in 70ml ethanol.

The resulting solution was spray dried using a Buchi mini B-290 system. The Inlet temperature was set at 150°C and the pump rate adjusted to approximately 3.6 ml/min.

The product was recovered as a dry white powder which was redispersed in water to a concentration of 1 mg/ml and the particle size was measured using a Malvern Nano-S and found to be 94 nm (Zave)

Further Aspects of the Invention include:

### Aspect 1

A process for making contra-soluble nano-dispersions of at most sparingly-soluble materials in a soluble carrier material comprising the steps of:
(i) providing a single phase mixture of:
   (a) a solvent or a mixture of miscible solvents,
   (b) at least one carrier material soluble in solvent (a), said carrier material being also contra-soluble to payload material (c) and solid at ambient temperature,
   (c) at least one payload material which is soluble in solvent (a), and,
(ii) drying the mixture to remove solvent (a) and thereby obtain the carrier material (b) in solid form with payload (c) dispersed therein as nanoparticles.

### Aspect 2

A process according to Aspect 1 wherein the solvent (a) comprises one or more of dichloromethane, chloroform, methanol, ethanol, isopropanol, isobutanol, formic acid, acetic acid, formamide, N,N-dimethylformamide, aceto-nitrile, ethyl acetate, methyl ethyl ketone, acetone, tetrahydrofuran, and dialkylsulphoxide.

### Aspect 3

A process according to Aspect 1 wherein the carrier (b) comprises one or more of Polyethylene glycol (PEG), Polyvinyl alcohol (PVA), polyvinylpyrrolidone (PVP), hydroxypropyl cellulose, hydroxypropyl methyl-cellulose (HPMC), alginate PEG/PPG alkoxylated non-ionic surfactant, PEG/alcohol non-ionic surfactant, aromatic non-ionic surfactant, alkyl sulphonate surfactant, ether-sulphate surfactants, ester surfactant and cationic surfactant.

### Aspect 4

A process according to Aspect 1 wherein the carrier (b) comprises:
(a) at least one of: polyethylene glycol (PEG), polyvinylpyrrolidone (PVP), poly(2-ethyl-2-oxazaline), polyvinyl alcohol (PVA) hydroxypropyl cellulose and hydroxypropyl-methyl cellulose (HPMC), alginate, and,
(b) at least one of: alkoxylated nonionic's, phenol-ethoxylates, alkyl sulphonates (preferably SDS), ether-sulphates (preferably SLES), ester surfactants, cationics (preferably cetyltrimethylammonium bromide - CTAB).

### Aspect 5

A process according to any of Aspects 1 to 4, for making a nano-dispersion of a water insoluble material in a water-soluble carrier material comprising the steps of:
(i) providing a mixture of:
   (a) an aqueous solvent for a carrier material;
   (b) at least one water-soluble carrier material soluble in the mixture of aqueous solvent (a) and second solvent (c), said carrier material being solid at ambient temperature,
   (c) at least one non-aqueous second solvent said second solvent being miscible with aqueous solvent (a) to form a mixed solvent capable of dissolving carrier material (b) and payload (d), and;
   (d) a payload material which is dispersible or soluble in the mixture of aqueous solvent (a) and non-aqueous solvent (c) but not in aqueous solvent (a) alone, and;
(ii) drying the mixture at above ambient temperature to remove solvents (a) and (c) and thereby obtain the carrier material (b) in a solid form with payload (d) dispersed therein in a nanoparticulate form,
wherein the product of the drying step is water dispersible to give an aqueous solution of (b) and an aqueous nano dispersion of (d).

### Aspect 6

A process according to any of Aspects 1 to 4, for making water-soluble nano-dispersions of water insoluble materials in a water-soluble carrier material comprising the steps of:
(i) providing a mixture of:
   (a) a non-aqueous solvent or a mixture of miscible non-aqueous solvents,
   (b) at least one carrier material soluble in non-aqueous solvent (a), said carrier material being also soluble in water and solid at ambient temperature,
   (c) at least one water-insoluble payload material which is soluble in non-aqueous solvent (a), and,
(ii) drying the mixture above ambient temperature to remove solvent (a) and thereby obtain the carrier material (b) in solid form with payload material (c) dispersed therein in a nanoparticulate form,
wherein the product of the drying step is water dispersible to give an aqueous solution of (b) and an aqueous nano dispersion of (c).

### Aspect 7

A process according to any one of Aspects 1 to 6 wherein the drying step comprises spray drying.

### Aspect 8

A process according to any one of Aspects 1 to 7 wherein the redispersed product of the drying step has a particle size of 500-2nm (expressed as a diameter) when measured using a Malvern 'Nano-S' apparatus.

### Aspect 9

A process according to any one of Aspects 1 to 8 wherein the payload material has a water solubility of below 5g/L.

### Aspect 10

A process according to any one of Aspects 1 to 9 wherein the feedstock to the drying step has a solids content of less than 5%wt.

### Aspect 11

A process according to any one of Aspects 1 to 10 wherein the product of the drying step comprises payload material of which at least 50%wt is in a crystalline form.

## Claims

1. A process for making water-soluble nano-dispersions of water-insoluble materials in a water-soluble carrier material comprising the steps of:
(i) providing a single phase mixture of:
a) a non-aqueous solvent or a mixture of miscible non-aqueous solvents,
b) at least one carrier material soluble in non-aqueous solvent (a), said carrier material being also soluble in water and solid at ambient temperature,
c) at least one water-insoluble payload material which is soluble in non-solvent (a), and,
(ii) drying the mixture to remove solvent (a) and thereby obtain the carrier material (b) in solid form with payload (c) dispersed therein as nanoparticles, wherein the product of the drying step is water dispersible to give an aqueous solution of (b) and an aqueous nano-dispersion of (c).

2. A process according to claim 1 wherein the solvent (a) comprises one or more of dichloromethane, chloroform, methanol, ethanol, isopropanol, isobutanol, formic acid, acetic acid, formamide, N,N-dimethylformamide, aceto-nitrile, ethyl acetate, methyl ethyl ketone, acetone, tetrahydrofuran, and dialkylsulphoxide.

3. A process according to claim 1 wherein the carrier (b) comprises one or more of Polyethylene glycol (PEG), Polyvinyl alcohol (PVA), polyvinylpyrrolidone (PVP), hydroxypropyl cellulose, hydroxypropyl methyl-cellulose (HPMC), alginate PEG/PPG alkoxylated non-ionic surfactant, PEG/alcohol non-ionic surfactant, aromatic non-ionic surfactant, alkyl sulphonate surfactant, ether-sulphate surfactants, ester surfactant and cationic surfactant.

4. A process according to claim 1 wherein the carrier (b) comprises:
a) at least one of: polyethylene glycol (PEG), polyvinylpyrrolidone (PVP), poly(2-ethyl-2-oxazaline), polyvinyl alcohol (PVA) hydroxypropyl cellulose and hydroxypropyl-methyl cellulose (HPMC), alginate, and,
b) at least one of: alkoxylated nonionic's, phenol-ethoxylates, alkyl sulphonates (preferably SDS), ether-sulphates (preferably SLES), ester surfactants, cationics (preferably cetyltrimethylammonium bromide - CTAB).

5. A process according to any one of claims 1-4 wherein the drying step comprises spray drying.

6. A process according to any one of claims 1-5 wherein the redispersed product of the drying step has a particle size of 500-2nm (expressed as a diameter) when measured using a Malvern™ 'Nano-S' apparatus.

7. A process according to any one of claims 1-6 wherein the payload material has a water solubility of below 5g/L.

8. A process according to any one of claims 1-7 wherein the feedstock to the drying step has a solids content of less than 5%wt.
